# EUROPÄISCHE PATENTSCHRIFT

(11) **EP 2 528 963 B1**
(45) Veröffentlichungstag und Bekanntmachung des Hinweises auf die Patenterteilung: **23.09.2015**
(21) Anmeldenummer: 11701967.9
(22) Anmeldetag: 24.01.2011
(51) Int. Cl.: C08G 63/16, C08G 69/26

(54) **POLYMERE AUS NACHWACHSENDEN ROHSTOFFEN**
POLYMERS MADE OF RENEWABLE RESOURCES
POLYMÈRES CONSTITUÉS DE MATIÈRES PREMIÈRES RENOUVELABLES

(30) Priorität: 25.01.2010 DE 102010005770
(43) Veröffentlichungstag der Anmeldung: 05.12.2012
(73) Patentinhaber: BASF SE, 67056 Ludwigshafen (DE)
(72) Erfinder: QUINZLER, Dorothee, 78462 Konstanz (DE); MECKING, Stefan, 78464 Konstanz (DE)
(86) Internationale Anmeldenummer: PCT/EP2011/050924
(87) Internationale Veröffentlichungsnummer: WO 2011/089256

(56) Entgegenhaltungen:
- JIMENEZ-RODRIGUEZ ET AL: "Dicarboxylic acid esters from the carbonylation of unsaturated esters under mild conditions", INORGANIC CHEMISTRY COMMUNICATIONS, ELSEVIER, AMSTERDAM, NL, Bd. 8, Nr. 10, 1. Oktober 2005 (2005-10-01), Seiten 878-881, XP005077103, ISSN: 1387-7003, DOI: DOI:10.1016/J.INOCHE.2005.06.005
- J. Falbe, M. Regitz: "Römpp Chemie Lexikon", 1990, Georg Thieme Verlag, Stuttgart, XP002638485, ISBN: 3-13-734709-2 Seite 1223

## Beschreibung

Die Erfindung betrifft Polykondensate mit längeren linearen Methylensequenzen in der Hauptkette, Verfahren zu deren Herstellung, deren Verwendung und ein katalytisches Verfahren zur Herstellung der Monomere.

Nachwachsende Rohstoffe sind als Alternative zu endlichen fossilen Rohstoffen von Interesse. Zudem können damit neuartige Materialien mit vorteilhaften Eigenschaften zugänglich sein. Hierfür ist es wünschenswert, dass sich die molekulare Struktur des Rohstoffes auch in der Struktur der resultierenden Produkte wiederspiegelt.

Ein attraktives Charakteristikum von Pflanzenölen und Fetten ist die lineare Kohlenwasserstoff-Struktur der (in Form der Triglyceride) enthaltenen Fettsäuren. Diese wird seit langem für die Erzeugung des technischen Kunststoffs Polyamid-11 ausgehend von Rizinolsäure genutzt. In diesem Fall wird allerdings nur ein Teil des Fettsäuremoleküls genutzt. Die thermische Umlagerung von Rizinolsäure führt unter Spaltung der Fettsäurekette zu Undec-10-ensäure, welche weiter zum Polyamid-11 umgesetzt wird, sowie Heptanal als Nebenprodukt. Zudem erfordert die zugrundeliegende Umlagerungsreaktion die Anwesenheit der Hydroxygruppe in Nachbarschaft zur Doppelbindung und ist daher unter den derzeit technisch verfügbaren Fettsäuren auf Rizinolsäure und deren Derivate beschränkt. Aus Rizinolsäure ist auch Sebacinsäure zugänglich, welche zu Polykondensaten wie Polyamid-6,10 umgesetzt wird. In diesem Fall entsteht als Nebenprodukt 2-Octanol.

Ein vollständiger linearer Einbau von langkettigen C_{>20} Fettsäuren in Polymere ist wünschenswert, da hiermit zum einen das Substrat vollständig stofflich zum Aufbau der Polymere genutzt würde, zum anderen lange lineare Methylensequenzen zu wünschenswerten Eigenschaften führen können, wie Kristallinität und damit verbundenen Schmelz- und Kristallisationspunkten, mechanischen und sonstigen Anwendungseigenschaften, Mischungsverhalten in Polymerblends, oder einer geringen Wasseraufnahme. Durch Dimerisierung können Fettsäuren zu den sogenannten Dimerfettsäuren umgesetzt werden, welche aber eine verzweigte, nicht-lineare Struktur aufweisen und zu nicht-kristallinen Segmenten führen (Angew. Chem. 2000, 112, 2292). Ein vollständiger linearer Einbau von Fettsäuren in Monomere und daraus erzeugte Polymere ist schwierig zu realisieren, da sich die reaktive Doppelbindung ungesättigter Fettsäuren in der Mitte des Moleküls befindet und die terminale Methylgruppe wenig reaktiv ist. Bei der Umsetzung von Rohstoffen aus nachwachsenden Quellen ist generell zu bedenken dass diese meist Verunreinigungen enthalten, welche Umsetzungsverfahren stören können und zu Produktgemischen anstelle von Reinsubstanzen führen. Unterschiedliche Fettsäuren oder deren Derivate weisen beispielsweise je nach biologischer Herkunft häufig ein unterschiedliches Spektrum von Verunreinigungen auf.

Zudem führen die meisten Syntheseverfahren für längerkettige α,ω-Dicarbonsäuren oder deren Derivate, unabhängig von ihrer Praktikabilität und der Beschaffenheit und Verfügbarkeit der Ausgangssubstanzen, zu den geradzahligen Produkten.

Die katalytische Alkoxycarbonylierung von Olefinen, das heißt die Umsetzung mit Kohlenmonoxid und einem Alkohol, ist eine bekannte Reaktion zur Erzeugung von Estern (Applied Homogeneous Catalysis with Oiganornaetallic Compounds, Hrsg. B. Cornils und W. A. Herrmann, Wiley-VCH, Weinheim, 2000). So führt die Cobalt-katalysierte Umsetzung von Methylestern einfach ungesättigter Fettsäuren unselektiv zu 60:40 Gemischen der linearen α,ω-Dicarbonsäuredimethylester und verzweigten Diestern (Fette, Seifen, Anstrichmittel 1985, 87, 403). Für die Methoxycarbonylierung von Ethylen wurde gefunden, dass ein Palladium(II)-Katalysator modifiziert durch 1,2-*bis*[(di-tert.-butylphosphino)methyl]benzol besonders gut geeignet ist (Chem. Commun. 1999, 1877). Bemerkenswerterweise setzen diese Katalysatoren auch interne Olefine, wie 4-Octen, zu den linearen Methylestern um (Chem. Commun. 2004, 1720). Die analoge Umsetzung in methanolischer Lösung des intern ungesättigten C₁₈-Fettsäuremethylesters Methyloleat, sowie der mehrfach ungesättigten Analoga Methyllinolat und Methyllinolenat, führt bevorzugt zu dem gesättigten α,ω-Dicarbonsäureester, 1,19-Dimethylnonadecandioat (Inorg. Chen. Commun. 2005, 8, 878). Allerdings wurde dieser nicht in der Reinheit erhalten, wie sie für die Erzeugung von Polykondensaten erforderlich ist (Trans. Faraday Soc. 1936, 32, 39).

Erucasäure ist ein Beispiel für eine ungesättigte gut verfügbare C_{>20} Fettsäure. Die Umsetzung von Methylerucat mit Kohlenmonoxid in Methanol mit Trialkyl-Diphosphin-modifiziertem Palladium(II)-Katalysator erwies sich als problematisch, es wurde keine zufriedenstellende Alkoxycarbonylierung beobachtet. Überraschend wurde aber gefunden, dass mit Erucansäure und anderen ungesättigten Fettsäuren mit mehr als zwanzig C-Atomen (C_{>20} Fettsäuren) in höheren Alkoholen die Umsetzung selektiv zu α,ω-Dicarbonsäureestern gelingt und diese in Reinheiten von > 99% erhalten werden.

Weiterhin wurde gefunden, dass sich die entsprechenden α,ω-Dicarbonsäuren und deren Ester, sowie ihre reduzierten Hydroxy- und Aminoderivate in hervorragender Weise als Monomere zur Herstellung von Polykondensaten eignen.

Gegenstand der Erfindung ist daher ein Polykondensat, enthaltend eine oder mehrere Wiederholeinheiten der Formel (I),

-Z-(CH₂)₂ₙ₊₁-Z^{'}- (I),

worin die Symbole und Indizes folgende Bedeutungen haben:
- Z, Z': ist gleich oder verschieden -X-C(=O)∼, -C(=O)-HN-CH₂∼, -C(=O)-O-CH₂∼, -NH-C(=O)-O-CH₂∼ -O-C(=O)-NH-CH₂∼;
- X: ist O oder NH;
- ∼: gibt die Bindung an die Gruppe (CH₂)₂ₙ₊₁ an und
- n: ist eine Zahl ≥ 10.

Weiterhin Gegenstand der Erfindung ist ein Verfahren zur Herstellung des erfindungsgemäßen Polykondensats, wobei mindestens eine monomere Verbindung der Formel (II),

Z¹-(CH₂)₂ₙ₊₁-Z^{1'} (II)

worin
- Z¹, Z^{1'}: ∼C(=O)-Q oder ∼CH₂-NCO ist;
- Q: gleich oder verschieden OH, Halogen oder C₁-C₁₀-Alkoxy ist;
- ∼: die Bindung an die Gruppe (CH₂)₂ₙ₊₁ angibt und
- n: eine Zahl ≥ 10 ist,
mit mindestens einem Di- oder Polyol oder mindestens einem Di- oder Polyamin polykondensiert wird,
und/oder
mindestens eine monomere Verbindung der Formel (III),

Z²-(CH₂)₂ₙ₊₁-Z^{2'} (III),

worin
- Z², Z²': gleich oder verschieden HO-CH₂∼ oder H₂N-CH₂∼ ist
- ∼: die Bindung an die Gruppe (CH₂)₂ₙ₊₁-Gruppe angibt und
- n: eine Zahl ≥ 10 ist,
mit mindestens einer Di- oder Polycarbonsäure, einem reaktiven Derivat einer solchen Di- oder Polycarbonsäure oder mindestens einem Di- oder Polyisocyanat polykondensiert.

Weiterhin Gegenstand der Erfindung ist die Verwendung des erfindungsgemäßen Polykondensats in Formteilen, Beschichtungen, Schäumen, Filmen, Folien und/oder Fasern, sowie die entsprechenden Gegenstände, welche das erfindungsgemäße Polykondensat enthalten.

Ebenso Gegenstand der Erfindung ist ein Verfahren zur Herstellung von gesättigten oder ungesättigten α,ω-Dicarbonsäuren oder Estern mit jeweils 2n+3 Kohlenstoffatomen im Säureteil, wobei n≥10 ist, umfassend den Schritt einer Hydroxy- oder Alkoxycarbonylierung eines einfach oder mehrfach ungesättigten Fettsäureesters mit 2n+2 Kohlenstoffatomen im Säureteil, wobei n≥10 ist, in Gegenwart eines Katalysators, enthaltend mindestens eine Palladiumverbindung und mindestens ein Phosphan bei einer Temperatur im Bereich von 50 bis 120 °C und einem Druck von 3 bis 80 atm.

Die Monomere zur Herstellung der erfindungsgemäßen Polykondensate werden erfindungsgemäß durch Hydro- oder Alkoxycarbonylierung ungesättigter Fettsäuren mit mindestens 22 C-Atomen erhalten. Die Umsetzung erfolgt in Gegenwart eines Katalysators.

Der verwendete Katalysator ist dadurch gekennzeichnet, dass er eine Palladiumverbindung und mindestens einen phosphorhaltigen Liganden enthält, welcher über eines oder mehrere Phosphoratome am Metallzentrum koordinieren kann. In dem phosphorhaltigen Liganden sind als Substituenten R"¹-R"³ am Phosphoratom PR"¹R"²R"³, unabhängig voneinander gleich oder verschieden H, offenkettige oder cyclische aliphatische Reste C₁ bis C₃₀, aromatische Reste C₁ bis C₃₀, insbesondere C₅-C₁₂, sowie über Heteroatome wie N, O oder S gebundene offenkettige oder cyclische aliphatische Reste C₁ bis C₃₀ und aromatische Reste C₁ bis C₃₀, insbesondere C₅-C₁₂, geeignet. Die Reste R"¹ bis R"³ können auch in der Seitenkette Heteroatome enthalten, wie beispielsweise O, N, S oder P. Besonders geeignete Reste sind tert.-Butylgruppen. Der Ligand kann mehrere Phosphoratome enthalten, welche über einen oder mehrere der Reste R" verbrückt sind. Besonders geeignete verbrückende Reste sind solche, welche die Phosphoratomen über drei oder vier Atome verbrücken. Besonders geeignete verbrückende Reste sind -CH₂-C₆H₄-CH₂- und -(CH₂)₃-.

Bevorzugt werden Phosphane verwendet. Besonders bevorzugt werden Trialkylphosphane und zweizähnige Phosphane verwendet. Beispiele für geeignete phosphorhaltige Liganden sind: 1,3-Bis(di-tert-butylphosphino)propan; 1,3-Bis(di-tert-butylphosphino)butan; 1,3-Bis(di-tert-butylphosphino)-2,2'-dimethyl-propan; 1,2-Bis[(di-tert-butylphosphino)methyl]benzol; 1,2-Bis[(cyclooct-1,5-diylphosphino)methyl]benzol; 1,3-Bis(cyclooct-1,5-diylphosphino)propan; 1-[(di-tert-butylphosphino)methyl]-2-(di-tert-butylphosphino)benzol.

Es kann eine Sorte phosphorhaltiger Ligand oder ein Gemisch von mehreren phosphorhaltigen Liganden verwendet werden.

Beispiele für geeignete Palladiumverbindungen sind Palladiumacetat, Palladiumhexanoat, Palladiumoctanoat, Bis(dibenzylidenaceton)palladium, Tetrakis(triphenylphosphan)palladium, (1,5-Cyclooctadien)dimethylpalladium, (1,5-Cyclooctadien)dichloropalladium, (1,5-Cyclooctadien)methylchloropalladium, Tetrakis(acetonitril)palladium(II)tetrafluoroborat und Diacetonitrildichloropalladium.

Der Katalysator enthält optional weitere Komponenten, beispielsweise organische und/oder anorganische Säuren und/oder deren Salze. Als geeignete weitere Komponenten seien beispielhaft genannt Trifluormethansulfonsäure, Methansulfonsäure, Perchlorsäure, p-Toluolsulfonsäure, [H(OEt₂)][BAr^{F}₄], NaBAr^{F}₄ mit Ar^{F} = 3,5-Bis(trifluormethyl)phenyl.

Die Katalysatorkomponenten können in beliebiger Folge miteinander gemischt werden. Die Mischung kann in oder ausserhalb des Reaktionsgefäßes der Carbonylierung erfolgen. Insbesondere können vorgebildete Palladium-Komplexe des Phosphans eingesetzt werden. Beispiele sind [(Diphosphin)PdX₂] mit X = Methansulfonat, Trifluormethansulfonat, oder Tosylat.

Als Katalysator besonders geeignet ist eine Kombination eines Palladium(II)-Salzes und 1,2-Bis[(di-tert.-butylphosphino)methyl]benzol, insbesondere in Kombination mit Methansulfonsäure.

Das molekulare Verhältnis von Palladium(II)-Verbindung zu Phosphor im Phosphanligand beträgt im Allgemeinen von 1 : 0,5 bis 1 :100, vorzugsweise von 1 : 0,5 bis 1 : 20, besonders bevorzugt von 1 : 2 bis 1 : 10.

Das molekulare Verhältnis von Pd(II)-Verbindung zu der optional vorhandenen Säure beträgt gegebenenfalls von 1 : 0,5 bis 1 : 200.

Als Reaktionsmedium eignen sich die für die Alkoxycarbonylierung benötigten Alkohole. Bevorzugt sind C₂-C₃₆ Alkohole, wie Ethanol, n-Propanol, i-Propanol, n-Butanol, i-Butanol, t-Butanol, Pentanol, Hexanol, Heptanol, n-Octanol, i-Octanol, 2-Ethylhexanol, Cyclohexanol, 2-Phenylethanol, Phenol, und Benzylalkohol oder Mischungen. Zusätzlich geeignet sind aprotische organische Lösungsmittel wie Methylenchlorid, Chloroform, Dichlorethan, Benzol, Toluol, Chlorbenzol, Isobutan, Hexan, Heptan, Octan, Diethylether, Diphenylether, Tetrahydrofuran, 2-Methyltetrahydrofuran, Sulfolan, N-Methylpyrollidon, Dimethylacetamid, Aceton, Cyclohexanon, Triethylamin, Tributylamin sowie Gemische dieser Verbindungen. Das Reaktionsmedium kann im Allgemeinen bis zu 50 vol.-% Methanol enthalten, bevorzugt weniger als 20 vol.-% Methanol. Das Reaktionsgemisch kann wasserfrei sein oder Wasser enthalten. Zur Herstellung der erfindungsgemäß eingesetzten α,ω-Dicarbonsäuren durch Hydrocarbonylierung ist die Anwesenheit von Wasser notwendig. Wird zur Herstellung von α,ω-Diestern eine Alkoxycarbonylierung durchgeführt, ist das Reaktionsgemisch bevorzugt wasserfrei.

Weiterhin kann auch der eingesetzte Fettsäureester als Reaktionsmedium dienen. Das Reaktionsgemisch kann ein- oder mehrphasig sein. Bevorzugt besteht das Reaktionsgemisch aus nur einer flüssigen Phase. Während der Reaktion können optional Reaktionsmedium, Reagenzien und Katalysator oder Katalysatorkomponenten kontinuierlich oder gepulst zugegeben werden.

Die Temperatur des Reaktionsgemisches liegt im Bereich von -10 °C bis +200 °C, bevorzugt im Bereich von +50 °C bis 120 °C, besonders bevorzugt im Bereich von 85 °C bis 100 °C. Der Druck während der Reaktion ist im Bereich von 0.1 bis 200 atm, bevorzugt 3 bis 80 atm, besonders bevorzugt 10 bis 40 atm.

Als Substrat werden Ester von ein- oder mehrfach ungesättigten Fettsäuren mit einer geraden Anzahl von Kohlenstoffatomen im Säureteil, die größer 20 und vorzugsweise nicht größer als 34 ist, verwendet. Das Symbol n in den Formeln steht daher für eine natürliche Zahl. Bevorzugt sind ein- oder mehrfach, besonders bevorzugt einfach ungesättigte Fettsäureester mit 22, 24, 26, 28, 30, 32 oder 34 C-Atomen (im Säureteil), besonders bevorzugt 22, 24 oder 26 C-Atomen, insbesondere 22 C-Atomen. Gegebenenfalls können auch die freien Säuren eingesetzt werden. Als Ester dieser Fettsäuren sind Ester monofunktioneller Alkohole bevorzugt, besonders bevorzugt C₁-C₁₀-Alkylester.

Besonders geeignet sind Ester der Erucasäure (cis-13-Docosensäure), insbesondere der Ethylester.

Als Substrate eingesetzt werden können Ester reiner C_{>20}-Fettsäuren oder technische C_{>20}-Fettsäure-Ester, welche unter anderem mehrfach ungesättigte Fettsäureester enthalten. Obwohl Ester monofunktionaler Alkohole bevorzugt sind, ist auch der Einsatz von Estern polyfunktionaler Alkohole, beispielsweise von Triglyzeriden, möglich.

Bevorzugt ist der Einsatz von Fettsäuren und deren Estern aus natürlichen Quellen, beispielsweise aus Pflanzenölen. Insbesondere bevorzugt zur Gewinnung von Estern der Erucasäure sind die Samen von erucasäurehaltigen Raps- und Meerkohlsorten, beispielsweise von Abessinischem Meerkohl.

Das molare Verhältnis des Fettsäureesters, bevorzugt Erucasäureesters, zu der molaren Menge Palladium in dem erfindungsgemäß eingesetzten Katalysator beträgt im Allgemeinen 10.000.000 : 1 bis 10 : 1, bevorzugt 1.000.000 : 1 bis 100 : 1, besonders bevorzugt 10.000 : 1 bis 500 : 1.

Die Carbonylierung von C_{>20} Fettsäureestern führt bei Einsatz von Estern geradzahliger Fettsäuren zu Estern ungeradzahliger linearer C_{>21} α,ω-Dicarbonsäuren.

Die erfindungsgemäß hergestellten α,ω-Dicarbonsäuren und deren Ester eignen sich als Monomere zur Herstellung erfindungsgemäßer Polykondensate.

Weiterhin geeignet sind reaktive Derivate dieser Säuren wie Säurehalogenide, aktivierte Ester, Anhydride oder Imidazolide.

Ebenfalls geeignet, beispielsweise in Kombination mit den Disäuren beziehungsweise Diestern, sind die entsprechenden α,ω-Diole, welche aus den Disäuren/Diestern nach bekannten, dem Fachmann geläufigen Reduktionsverfahren erhalten werden.

Unter Anwendung bekannter Methoden zur Reduktion von Carbonsäurederivaten zu Alkoholen konnte zum Beispiel 1,23-Diethyltricosandioat zu > 99 % reinem 1,23-Dihydroxytricosan umgesetzt werden. Beispiele für geeignete Methoden zur Reduktion von Carbonsäuren oder deren Derivaten zu Alkoholen sind die katalytische Hydrierung mit Wasserstoff, die Reduktion durch anorganische hydridische Reagenzien, und die Reduktion durch organische Verbindungen unter Oxidation derselben.

Weiterhin lassen sich die erfindungsgemäß hergestellten α,ω-Dicarbonsäuren und deren Ester, wie auch die daraus erhältlichen α,ω-Diole, nach bekannten, dem Fachmann geläufigen Methoden in die entsprechenden α,ω-Diamine umwandeln, die wiederum als Monomere bei der Synthese erfindungsgemäßer Polykondensate Anwendung finden können. Beispielhaft genannte bekannte Verfahren zur Umsetzung von Carbonsäuren oder deren Estern zu Aminogruppen sind Amidierung, gefolgt von Wasserabspaltung, und Hydrierung; zur Umsetzung von Alkoholen zu Aminen Halogenierung oder Tosylierung, gefolgt von Umsetzung mit Ammoniak oder alternativ Umsetzung zum Azid gefolgt von Reduktion. Zur Erzeugung von Diisocyanaten können die Amine mit Phosgen oder Phosgenäquivalenten umgesetzt werden.

Ester linearer ungeradzahliger C_{≥23} aliphatischer α,ω-Dicarbonsäuren, und lineare ungeradzahlige C_{≥23} aliphatische α,ω-Diole können zu Polymeren umgesetzt werden, welche ein Gegenstand der Erfindung sind. Die Diester können dabei als solche oder in aktivierter Form, beispielsweise als Säuren, als Chloride, oder Anhydride, eingesetzt werden..; Bevorzugt werden zur Umsetzung zu Polymeren 1,23-Tricosandicarbonsäure oder deren Ester, 1,23-Tricosandiol, 1,23-Tricosandiamin und 1,23-Tricosandiisocyanat eingesetzt. Besonders bevorzugt wird 1,23-Tricosandicarbonsäure oder deren Ester eingesetzt.

Neben mindestens einer der im vorangehenden Absatz ausgeführten erfindungsgemäß erhältlichen α,ω-difunktionellen Verbindungen können bei der Herstellung der erfindungsgemäßen Polymere auch weitere mehrfach funktionelle Alkohole, Amine, Säuren oder deren Ester, Chloride oder Anhydride, cyclische Lactame oder Lactone sowie Isocyanate eingesetzt werden. Diese können neben den genannten funktionellen Gruppen auch weitere Heteroatome enthalten. Beispiele für geeignete Alkohole sind Ethylenglycol, 1,3-Propandiol, 1,2-Propandiol, 1,4-Butandiol, Cyclohexandiol, 1,6-Hexandiol, 1,10-Decandiol, 1,12-Dodecandiol, 1 , 18-Octadecandiol, 1,19-Nonadecandiol, 2,2-Di(4-Hydroxyphenyl)propan, Diethylenglykol, Dihydroxy-terminiertes Polyethylenglykol und Trimethylolpropan sowie aromatische Di- und Polyhydroxyverbindungen, wie 4,4'-Dihydroxybiphenyl und Bisphenole wie Bisphenol A und Bisphenol AF. Beispiele für geeignete Amine sind Ethylendiamin, Diethylentriamin, 1,2-Diaminopropan, 1,3-Diaminopropan, 1,4-Diaminobutan, 1,5-Diaminopentan, 1,6-Diaminohexan, 1,10-Diaminodecan, 1,12-Diaminododecan sowie aromatische Diamine wie p-Phenylendiamin. Beispiele für geeignete Carbonsäuren sind Maleinsäure, Bernsteinsäure, Fumarsäure, Adipinsäure, Sebacinsäure, 1,19-Nonadecandisäure, Phthalsäure, Terephthalsäure. Beispiele für geeignete Lactame sind ε-Caprolactam, Lauryllactam. Beispiele für geeignete Lactone sind ε-Caprolacton und Butyrolacton. Beispiele für geeignete Diisocyanate sind Hexamethylendiisocyanat und 4,4'-Diphenylmethandiisocyanat.

Bevorzugt wird 1,23-Tricosandicarbonsäure oder deren Ester von monofunktionellen Alkoholen, in Gegenwart oder Abwesenheit weiterer der im vorangehenden Absatz beispielhaft genannten Dicarbonsäuren oder deren Estern, umgesetzt mit einem oder mehreren Diolen, Diaminen, optional in Gegenwart von Lactamen. Geeignete Diole, Diamine und Lactame sind im vorangehenden Absatz beispielhaft genannt.

Besonders bevorzugt wird 1,23-Tricosandicarbonsäure mit einer oder mehreren der folgenden Verbindungen polykondensiert: Ethylenglycol, 1,3-Propandiol, 1,4-Butandiol, Cyclohexandiol, 1,6-Hexandiol, 1,10-Decandiol, 1,12-Dodecandiol, 1,18-Octadecandiol, 1,19-Nonadecandiol, 1,23-Tricosandiol 2,2-Di(4-hydroxyphenyl)propan, Diethylenglykol, Dihydroxy-terminiertes Polyethylenglykol, Ethylendiamin, Diethylentriamin, 1,3-Diaminopropan, 1,4-Diaminobutan, 1,5-Diaminopentan, 1,6-Diaminohexan, 1,10-Diaminodecan, 1,12-Diaminododecan, 1,23-Tricosandiamin.

Ebenfalls besonders bevorzugt wird die erfindungsgemäß eingesetzte α,ω-Dicarbonsäure, vorzugsweise 1,23-Tricosandicarbonsäure, zusammen mit Bernsteinsäure, Adipinsäure und/oder Terephtalsäure mit einer oder mehreren der folgenden Verbindungen polykondensiert: Ethylenglycol, 1,3-Propandiol, 1,4-Butandiol, Cyclohexandiol, 1,6-Hexandiol, 1,10-Decandiol, 1,12-Dodecandiol, 1,18-Octadecandiol, 1,19-Nonadecandiol, 1,23-Tricosandiol, 2,2-Di(4-hydroxyphenyl)propan, Diethylenglykol, Dihydroxy-terminiertes Polyethylenglykol, Ethylendiamin, Diethylentriamin, 1,3-Diaminopropan, 1,4-Diaminobutan, 1,5-Diaminopentan, 1,6-Diaminohexan, 1,10-Diaminodecan, 1,12-Diaminododecan, 1,23-Tricosandiamin.

Polymerisationsverfahren sind dem Fachmann hinlänglich bekannt. Die Polymerisation kann in Anwesenheit oder Abwesenheit von Katalysatoren, Lösungsmitteln, Suspensionsmitteln oder anderen Zusatzstoffen erfolgen. Die Polymerisation kann beispielsweise in Lösung, Schmelze, Suspension, nichtwässriger oder wässriger Dispersion erfolgen. Geeignete Katalysatoren sind beispielsweise organische oder anorganische Säuren; sowie Alkoxide, Carboxylate und Oxide von Übergangs- oder Hauptgruppenmetallen. Die Polymerisation erfolgt bei 0.00001 mbar bis 100 bar Druck. Zur Entfernung flüchtiger Nebenprodukte kann die Polymerisation im Vakuum erfolgen. Das Polymerisationsgemisch kann zwangsgemischt werden, beispielsweise durch Rührer oder Extruder. Die Polymerisation kann einstufig oder mehrstufig erfolgen.

Optional kann während oder nach der Polymerisation mittels geeigneter Reagenzien Vernetzung erfolgen. Dadurch können bestimmte Eigenschaften, beispielsweise Elastizität oder Fließeigenschaften, erhalten oder verbessert werden.

Weiterhin können die Eigenschaften der erfindungsgemäßen Polykondensate durch Kettenverlängerung, beispielsweise durch Umsetzung mit Carbonylbiscaprolactam (CBC) und/oder Phenylen-1,4-bis-oxazolin (1.4-PBO), modifiziert werden. Weitere geeignete Methoden der Kettenverlängerung sind beispielsweise die Umsetzung mit mehrfach-funktionellen Isocyanaten. Die erfindungsgemäßen Polymere können als Präpolymere in Polykondensationen und Polyadditionen eingesetzt werden. Beispielsweise können dihydroxyterminierte erfindungsgemäße Polymere mit mehrfachfunktionellen Isocyanaten oder Carbonsäuren umgesetzt werden.

In einer bevorzugten Ausführungsform entsprechen die Gruppe Z, Z' in der Formel (I) -X-C(=O)- beziehungsweise -X'-C(=O)- wobei X, X' gleich oder verschieden, vorzugsweise gleich, O oder NH, vorzugsweise O, sind.

Diese Ausführungsform betrifft Polykondensate, enthaltend Wiederholeinheiten -X-C(=O)-(CH₂)₂ₙ₊₁-C(=O)-X'- mit n≥10 und X, X' O oder NH.

Weiterhin bevorzugt sind Polykondensate, in denen Z eine Gruppe -C(=O)-O-CH₂∼, -C(=O)-NH-CH₂∼, -NH-C(=O)-O-CH₂∼ oder -O-C(=O)-NH-CH₂∼ bedeutet, d.h. Polykondensate, enthaltend Wiederholeinheiten -M-(CH₂)₂ₙ₊₃-M'-, wobei n≥10 ist und M, M' eine Amid-, Ester- oder Urethanfunktion bedeuten.

Bevorzugt sind auch Copolyester, die neben einem erfindungsgemäßen Monomer weitere Monomere enthalten, bevorzugt Polyester [{-A¹-OC(=O)-(CH₂)₂ₙ₊₁-C(=O)O-}ₓ{-A¹-OC(=O)-A²-C(=O)O-}_{y}], wobei n ≥10 ist und A¹, A² einen C₂ bis C₃₆ aliphatischen, cycloaliphatischen, aromatischen oder gemischten Rest bedeuten. Ein gemischter Rest ist zum Beispiel eine araliphatische Gruppe. x und y bezeichnen jeweils den Molenbruch, d.h. x +y = 1.

Ebenfalls bevorzugt sind auch Copolyamide, die neben einem erfindungsgemäßen Monomer weitere Monomere aufweisen, insbesondere Polyamide [{-A³-NHC(=O)-(CH₂)₂ₙ₊₁-C(=O)NH-}ₓ{-A³-NHC(=O)-A⁴-C(=O)NH-}_{y}], wobei n ≥10 ist und A³, A⁴ jeweils einen aliphatischen, cycloaliphatischen, aromatischen oder gemischten Rest mit 2 bis 36 C-Atomen bedeuten.

Bevorzugt sind weiterhin erfindungsgemäße Polyester und Polyamide mit y = 0.

Weiterhin bevorzugt sind erfindungsgemäße Polykondensate mit n = 10, bei denen sich zumindest ein Teil der Monomere von 1,23-Tricosandicarbonsäure ableitet.

Die erfindungsgemäßen Polymere enthalten im Allgemeinen mindestens 1 mol-%, bevorzugt mindestens 10 mol-%, besonders bevorzugt 50 mol-% mindestens eines Typs der Wiederholeinheiten der Formel (I), bevorzugt der Wiederholeinheiten - X-C(=O)-(CH₂)₂ₙ₊₁-C(=O)-X'- oder -M-(CH₂)₂ₙ₊₃-M'-; mit n ≥ 10, wobei X,X' unabhängig voneinander gleich oder unterschiedlich O oder NH; M,M' unabhängig voneinander gleich oder verschieden eine Amid-, Ester- oder Urethanfunktion, bedeuten.

Die erfindungsgemäßen Polykondensate, insbesondere Polyester, weisen im Allgemeinen ein zahlenmittleres Molekulargewicht Mₙ von 1.000 bis 2.000.000 s/mol, bevorzugt von 5.000 bis 200.000 g/mol und besonders bevorzugt von 10.000 bis 50.000 g/mol auf.

Ein Vorteil der erfindungsgemäßen Polykondensate, insbesondere Polyester, ist zum einen, dass sie auf der Basis von erneuerbaren Materialien, zum Beispiel Erucasäureestern, basierend auf beispielsweise Meerkohl, hergestellt werden können. Ein weiterer Vorteil der erfindungsgemäßen Polykondensate, insbesondere Polyester, ist, dass sie grundsätzlich als bioabbaubare Polymere geeignet sind. Dabei werden unter bioabbaubaren Polymeren im Sinne der Erfindung solche Materialien bezeichnet, die durch Mikroorganismen, Enzyme oder Hydrolyse, zum Beispiel im Boden abgebaut werden. Die Prüfung der biologischen Abbaubarkeit erfolgt durch die DIN-Norm EN 13432. Die bioabbaubaren Materialien müssen innerhalb von 6 bis 10 Wochen in einer Großkompostierung abgebaut werden. In einer weiteren Ausführungsform betrifft die Erfindung somit einen erfindungsgemäßen Polyester, der bioabbaubar ist. Durch die langkettigen kristallinischen Segmente werden Schmelz- und Kristallisationseigenschaften in wünschenswerter Weise beeinflusst und sind ausreichend hoch für eine thermoplastische Verarbeitung. Erfindungsgemäße Polyester zeigen aufgrund ihrer Kristallinität entsprechend hohe Schmelz- und Kristallinitätspunkte, die beispielsweise bei Tₘ>80°C und T_{c}>70°C, insbesondere Tₘ>90°C und T_{c}>75°C, liegen können. Weiterhin zeigen die Systeme eine geringe Wasseraufnahme.

Die erfindungsgemäßen Polymere lassen sich in zahlreichen Anwendungen vorteilhaft verwenden, beispielsweise in Formteilen, Beschichtungen, Schäumen, Filmen, Folien und Fasern. Die erfindungsgemäßen Polymere können auch in Mischungen mit anderen Kunststoffen verwendet werden.

Als Verfahren zur Verarbeitung der erfindungsgemäßen Polymeres seien beispielhaft genannt Spritzguß, Coextrusion, Foliengiessen, Blasformen, Folienblasen, Kalandern, Schmelzpressen, Naßspinnen, Trockenspinnen, Schmelzspinnen, Tiefziehen, Pulverbeschichten und Beschichten aus organischer Lösung oder wässrigen Dispersionen.

Die Erfindung wird durch die Beispiele näher erläutert, ohne sie dadurch zu beschränken.

### Beispiele

### A Herstellung der Monomere

### Beispiel 1 Darstellung von 1,23 Diethyltricosandioat

Eine Mischung aus 10 mL Ethanol, 1.79 g Ethylerucat, 0.079 mmol Palladium(II)acetat, 0.39 mmol 1,2-*bis*[(di-tert.-butylphosphino)methyl]benzol und 0.79 mmol Methansulfonsäure wurde in einem Druckreaktor für 22 h bei 90 °C und 20 bar Kohlenmonoxiddruck gerührt. Das Reaktionsgemisch wurde am Rotationsverdampfer bei 200 mbar und 50 °C eingeengt. Anschliessend wurden 5 mL Dichlormethan zugesetzt. Die Mischung wurde filtriert, und am Rotationsverdampfer bei Normaldruck und 50 °C eingeengt Der Rückstand wurde aus Ethanol umkristallisiert. Ausbeute 76 % bezogen auf eingesetztes Ethylerucat. Laut ¹H NMR Spektrum handelt es sich zu > 99 % um 1,23-Diethyltricosandioat.

### (Vergleichsbeispiel)

Eine Mischung aus 10 mL Methanol, 1.79 g Methylerucat (95 %), 0.079 mmol Palladium(II)acetat, 0.39 mmol 1,2-*bis*[(di-tert.-butylphosphino)methyl]benzol und 0.79 mmol Methansulfonsäure wurde in einem Druckreaktor für 22 h bei 90 °C und 20 bar Kohlenmonoxiddruck gerührt. Das Gemisch enthält überwiegend den nicht-carbonylierten Monocarbonsäuremethylester.

### Beispiel 2 Darstellung von 1,23-Tricosandicarbonsäure

Zu einer Suspension aus 20 mL Methanol und 4.5 mmol 1,23-Diethyltricosandioat wurden insgesamt 55.2 mmol KOH zugefügt. Das Gemisch wurde unter Rühren 10 Stunden zum Rückfluss erhitzt. Anschließend wurde das Lösungsmittel im Vakuum entfernt. Zum erhaltenen weißen Feststoff wurden 30 mL Wasser gegeben und mit 6 N Salzsäure auf pH = 2 angesäuert. Die Mischung wurde filtriert und der weiße Feststoff aus Methanol umkristallisiert. Ausbeute 96 % bezogen auf eingesetztes 1,23-Diethyltricosandioat. Laut ¹H NMR Spektrum handelt es sich zu > 99 % um 1,23-Tricosandicarbonsäure.

### Beispiel 3 Darstellung von 1,23-Tricosandiol (Route A)

In 10 mL Tetrahydrofuran wurden 2.3 mmol 1,23-Diethyltricosandioat gelöst. Diese Lösung wurde langsam unter Rühren zu eine Suspension von 5.2 mmol Lithiumaluminiumhydrid in 10 mL Tetrahydrofuran zugetropft. Das Gemisch wurde unter Rühren für eine Stunde zum Rückfluss erhitzt, und anschließend über Nacht bei Raumtemperatur gerührt. Unter Rühren wurden langsam 0.2 mL Wasser, 0.2 mL 15%ige wässrige NaOH-Lösung, und 0.6 mL Wasser nacheinander zugegeben. Die Mischung wurde bei 40 °C filtriert. Das Filtrat wurde am Rotationsverdampfer bei 50 °C und 500 mbar eingeengt. Der Rückstand wurde aus Ethanol umkristallisiert. Ausbeute 80 % bezogen auf eingesetztes 1,23-Diethyltricosandioat. Laut ¹H NMR Spektrum handelt es sich zu > 99 % um 1,23-Dihydroxytricosan.

### Beispiel 4 Darstellung von 1,23-Tricosandiol (Route B)

Eine Mischung aus 40 mL Tetrahydrofuran, 5.6 mmol 1,23-Diethyltricosandioat, 5.6 µmol Dichlorobis[2-(diphenylphosphino)ethylamin]ruthenium und 0.56 mmol Natriummethanolat wurden in einem Druckreaktor für 22 Stunden bei 100 °C und 50 bar Wasserstoffdruck gerührt. Das Reaktionsgemisch wurde bei 40 °C filtriert. Das Filtrat wurde am Rotationsverdampfer bei 50 °C und 500 mbar eingeengt. Der Rückstand wurde aus Toluol umkristallisiert. Ausbeute 78 % bezogen auf eingesetztes 1,23-Diethyltricosandioat.

### B Polykondensationen und Polykondensate

### Beispiel 5 Polyester (23,23)

In einem 10 mL Kolben mit Hahn wurden 1.4 mmol 1,23-Diethyltricosandioat, 1.4 mmol 1,23-Dihydroxytricosan und 0.28 mmol Titantetrabutanolat im Laufe von 17 h von 110 °C auf 150 °C erwärmt bei 0.01 mbar. Nach dem Abkühlen wurde ein weißer Feststoff erhalten.

Gelpermeationschromatographie (Lösungsmittel 1,2,4-Trichlorbenzol, 160 °C, gegen lineare Polyethylen-Standards) ergab ein Molekulargewicht von Mₙ 10⁴ g mol⁻¹ (M_{w}/Mₙ 2). Differentielle Wärmeflußkalorimetrie (DSC) zeigt einen PeakSchmelzpunkt von Tₘ 99 ° in der ersten und zweiten Aufheizkurve, eine Kristallisationstemperatur von 84 °C, und eine Schmelzenthalpie von 180 J g⁻¹.

### Beispiel 6 Polyester (23,12)

In einem 100 mL Schlenk mit mechanischem Rührer wurden 2.8 mmol 1,23-Diethyltricosandioat und 1,12-Dodecandiol mit 0.028 mmol Titantetrabutanolat in 0.1 mL Toluol über einen Zeitraum von 8 Stunden von 100 °C auf 220°C erhitzt. Dann wurde bei 220 °C und 0.01 mbar 1 Stunde gerührt. Nach dem Abkühlen wurde ein weißer Feststoff erhalten.

Differentielle Wärmeflusskalorimetrie (DSC) zeigt einen Peakschmelzpunkt von Tₘ 101 °C in der zweiten Aufheizkurve, eine Kristallisationstemperatur von T_{c} 76 °C und eine Schmelzenthalpie von ΔHₘ 156 J g⁻¹.

### Beispiel 7 Polyester (23,6)

In einem 100 mL Schlenk mit mechanischem Rührer wurden 2.8 mmol 1,23-Diethyltricosandioat und 1,6-Hexandiol mit 0.028 mmol Titantetrabutanolat in 0.1 mL Toluol über einen Zeitraum von 8 Stunden von 100 °C auf 220°C erhitzt. Dann wurde bei 220 °C und 0.01 mbar 1 Stunde gerührt. Nach dem Abkühlen wurde ein weißer Feststoff erhalten.

Differentielle Wärmeflusskalorimetrie (DSC) zeigt einen Peakschmelzpunkt von Tₘ 92 °C in der zweiten Aufheizkurve, eine Kristallisationstemperatur von T_{c} 75 °C und eine Schmelzenthalpie von ΔHₘ 145 J/g.

### Beispiel 8 Polyester (23,4)

In einem 100 mL Schlenk mit mechanischem Rührer wurden 2.8 mmol 1,23-Diethyltricosandioat und 1,4-Butandiol mit 0.028 mmol Titantetrabutanolat in 0.1 mL Toluol über einen Zeitraum von 4 Stunden von 100 °C auf 200°C erhitzt. Dann wurde bei 220 °C und 8 Stunden gerührt und eine weitere Stunde bei 220 °C und 0.01 mbar. Nach dem Abkühlen wurde ein weißer Feststoff erhalten. Differentielle Wärmeflusskalorimetrie (DSC) zeigt einen Peakschmelzpunkt von Tₘ 85 °C in der zweiten Aufheizkurve, eine Kristallisationstemperatur von T_{c} 71 °C und eine Schmelzenthalpie von ΔHₘ 172 J g⁻¹.

### Beispiel 9 Polyamid (23,23)

Aus 1.04 mmol 1,23-Diaminotricosan und 1.04 mmol 1,23-Tricosandicarbonsäure wurde in 9 mL Ethanol Tricosamethylendiamintricosandioat hergestellt.

In einem 100 mL Schlenk mit mechanischem Rührer wurden 1.04 mmol Tricosamethylendiamintricosandioat mit 0.037 mmol 1,23-Diaminotricosan über einen Zeitraum von 3 Stunden von 120 °C auf 210°C unter Argonatmosphäre erhitzt und für 1.5 Stunden bei dieser Temperatur gerührt. Anschließend wurde bei 210 °C für 17.5 Stunden ein Vakuum von 1×10⁻⁵ bar angelegt. Nach dem Abkühlen wurde ein beiger Feststoff erhalten.

Differentielle Wärmeflusskalorimetrie (DSC) zeigt einen Peakschmelzpunkt von Tₘ 151 °C in der zweiten Aufheizkurve, eine Kristallisationstemperatur von T_{c} 130 °C, und eine Schmelzenthalpie von ΔHₘ 88 J g⁻¹.

### Beispiel 10 Polyamid (12,23)

Aus 1.43 mmol 1,12-Diaminododecan und 1.43 mmol 1,23-Tricosandicarbonsäure wurde in 9 mL Ethanol Dodecamethylendiamintricosandioat hergestellt.

In einem 100 mL Schlenk mit mechanischem Rührer wurden 1.43 mmol Dodecamethylendiamintricosandioat mit 0.045 mmol 1,12-Diaminododecan über einen Zeitraum von 5.5 Stunden von 100 °C auf 200°C unter Argonatmosphäre erhitzt und für 16.5 Stunden bei dieser Temperatur gerührt. Anschließend wurde bei 200 °C für 4 Stunden ein Vakuum von 1×10⁻⁵ bar angelegt. Nach dem Abkühlen wurde ein beiger Feststoff erhalten.

Differentielle Wärmeflusskalorimetrie (DSC) zeigt einen Peakschmelzpunkt von Tₘ 168 °C in der zweiten Aufheizkurve, eine Kristallisationstemperatur von T_{c} 150 °C und eine Schmelzenthalpie von ΔHₘ 121 J g⁻¹.

### Beispiel 11 Polyamid (23,19)

Aus 1.06 mmol 1,23-Diaminotricosan und 1.06 mmol 1,19-Nonadecandicarbonsäure wurde in 8mL Ethanol Tricosamethylendiaminnonadecandioat hergestellt.

In einem 100 mL Schlenk mit mechanischem Rührer wurden 1.06 mmol Tricosamethylendiaminnonadecandioat über einen Zeitraum von 4.5 Stunden von 100 °C auf 230°C unter Argonatmosphäre erhitzt und für 1.25 Stunden bei dieser Temperatur gerührt. Anschließend wurde bei 230 °C für 16.5 Stunden ein Vakuum von 1×10⁻⁵ bar angelegt. Nach dem Abkühlen wurde ein beiger Feststoff erhalten.

Differentielle Wärmeflusskalorimetrie (DSC) zeigt einen Peakschmelzpunkt von Tₘ 156 °C in der zweiten Aufheizkurve, eine Kristallisationstemperatur von T_{c} 132 °C und eine Schmelzenthalpie von ΔHₘ 85 J g⁻¹.

## Patentansprüche

1. Polykondensat, enthaltend eine oder mehrere Wiederholeinheiten der Formel (I),
-Z-(CH₂)₂ₙ₊₁-Z^{'}- (I)
worin die Symbole und Indizes folgende Bedeutungen haben:
Z, Z' ist gleich oder verschieden -X-C(=O)∼, -C(=O)-HN-CH₂∼, -C(=O)-O-CH₂∼, -NH-C(=O)-O-CH₂∼, -O-C(=O)-NH-CH₂∼;
X ist O oder NH;
∼ gibt die Bindung an die Gruppe (CH₂)ₙ an und
n ist eine Zahl ≥ 10.

2. Polykondensat gemäß Anspruch 1, enthaltend Wiederholeinheiten -X-C(=O)-(CH₂)₂ₙ₊₁-C(=O)-X'- mit n≥10 und X, X' = O oder NH.

3. Polykondensat gemäß Anspruch 1, enthaltend Wiederholeinheiten -M-(CH₂)₂ₙ₊₃-M'∼ wobei n ≥ 10 ist und M,M' eine Amid- (-C(=O)-NH∼), Ester-(-C(=O)-O∼) oder Urethanfunktion (-NH-C(=O)-O∼) bedeutet.

4. Polyester gemäß Anspruch 1 oder 2 mit Wiederholeinheiten [{-A¹-OC(=O)-(CH₂)₂ₙ₊₁-C(=O)O-}ₓ{-A¹-OC(=O)-A²-C(=O)O-}_{y}] wobei n ≥ 10 und A¹, A² gleich oder verschieden einen C₂ bis C₃₆ aliphatischen, cycloaliphatischen, aromatischen oder gemischten Rest bedeutet und x+y=1 gilt

5. Polyamid gemäß Anspruch 1 oder 2 mit Wiederholeinheiten [{-A1-NHC(=O)-(CH2) 2n+1-C(=O)NH-}x{-Al-NHC(=O)-A2-C(=O)NH-}y] wobei n ≥ 10 C₂ bis C₃₆ aliphatischen, cycloaliphatischen, aromatischen oder gemischten Rest bedeuten und x + y = 1 gilt.

6. Polykondensat gemäß Anspruch 3 oder 4, wobei y = 0 gilt.

7. Polykondensat gemäß einem der Ansprüche 1 bis 6 mit n = 10.

8. Polykondensat gemäß einem der Ansprüche 1 bis 7, enthaltend mindestens 1 mol-% an einer oder mehreren Wiederholeinheiten der Formel (I).

9. Verfahren zur Herstellung eines Polykondensats gemäß einem der Ansprüche 1 bis 8, wobei mindestens eine monomere Verbindung der Formel (II),
Z¹-(CH₂)₂ₙ₊₁-Z^{1'} (II),
worin
Z¹, Z^{1'} ∼C(=O)-Q oder ∼CH₂-NCO ist;
Q gleich oder verschieden OH, Halogen oder C₁-C₁₀-Alkoxy ist;
∼ die Bindung an die Gruppe (CH₂)₂ₙ₊₁ angibt und
n eine Zahl ≥ 10 ist,
mit mindestens einem Di- oder Polyol oder mindestens einem Di- oder Polyamin polykondensiert wird,
und/oder
mindestens eine monomere Verbindung der Formel (III),
Z²-(CH₂)₂ₙ₊₁-Z^{2'} (III),
worin
Z, Z' gleich oder verschieden HO-CH₂∼ oder H₂N-CH₂∼ ist
∼ die Bindung an die Gruppe (CH₂)ₙ-Gruppe angibt und
n eine Zahl ≥ 10 ist,
mit mindestens einer Di- oder Polycarbonsäure, einem reaktiven Derivat einer solchen Di- oder Polycarbonsäure oder mindestens einem Di- oder Polyisocyanat polykondensiert.

10. Verwendung eines Polykondensats gemäß einem der Ansprüche 1 bis 8 in Formteilen, Beschichtungen, Schäumen, Filmen, Folien und/oder Fasern.

11. Formkörper, Beschichtungen, Folien, Schäume, und Fasern enthaltend ein oder mehrere Polykondensate gemäß einem der Ansprüche 1 bis 8.

12. Verfahren zur Herstellung von gesättigten oder ungesättigten α,ω-Dicarbonsäuren oder Estern mit jeweils 2n+3 Kohlenstoffatomen im Säureteil, wobei n≥10 ist umfassend den Schritt einer Hydroxy- oder Alkoxycarbonylierung eines einfach oder mehrfach ungesättigten Fettsäureesters mit 2n+2 Kohlenstoffatomen im Säureteil, wobei n ≥10 ist, in Gegenwart eines Katalysators, enthaltend mindestens eine Palladiumverbindung und mindestens ein Phosphan, bei einer Temperatur im Bereich von 85 bis 100°C und einem Druck von 3 bis 80 atm.

13. Verfahren gemäß Anspruch 12, wobei die Alkoxyverbindung ein C₂-C₃₆-Alkohol ist.

14. Verfahren gemäß Anspruch 12 oder 13, wobei der Katalysator ein Palladium (Il)-Salz und 1,2-bis-[(di-tert.-Butylphosphino)methyl]benzol enthält.

15. Verfahren gemäß einem der Ansprüche 12 bis 14, wobei der Fettsäureester ein Ester der Erucasäure ist.

## Claims

1. A polycondensate comprising one or more repeat units of the formula (I),
-Z-(CH₂)₂ₙ₊₁-Z'- (I)
in which the symbols and indices have the following meanings:
Z, Z' is identical or different and is -X-C(=O)∼, -C(=O)-HN-CH₂∼, -C(=O)-O-CH₂∼, -NH-C(=O)-O-CH₂∼, -O-C(=O)-NH-CH₂∼;
X is O or NH;
∼ indicates the bond to the group (CH₂)ₙ and
n is a number ≥ 10.

2. The polycondensate according to claim 1, comprising repeat units -X-C(=O)-(CH₂)₂ₙ₊₁-C(=O)-X'-where n ≥ 10 and X, X' = O or NH.

3. The polycondensate according to claim 1, comprising repeat units -M-(CH₂)₂₊₃-M'∼ where n is ≥ 10 and M, M' is an amide function (-C(=O)-NH∼), ester function (-C(=O)-O∼) or urethane function (-NH-C(=O)-O∼).

4. The polyester according to claim 1 or 2 with repeat units [{-A¹-OC(=O)-(CH₂)₂ₙ₊₁-C(=O)O-}ₓ{-A¹-OC(=O)-A²-C(=O)O-}_{y}] where n ≥ 10 and A¹, A² are identical or different and are a C₂ to C₃₆ aliphatic, cycloaliphatic, aromatic or mixed radical and x + y = 1.

5. The polyamide according to claim 1 or 2 with repeat units [{-A1-NHC(=O)-(CH2)2n+1-C(=O)NH-}x{-A1-NHC(=O)-A2-C(=O)NH-}y] where n ≥ 10 are C₂ to C₃₆ aliphatic, cycloaliphatic, aromatic or mixed radical and x + y = 1.

6. The polycondensate according to claim 3 or 4, where y = 0.

7. The polycondensate according to any one of claims 1 to 6 where n = 10.

8. The polycondensate according to any one of claims 1 to 7, comprising at least 1 mol% of one or more repeat units of the formula (I).

9. A method for producing a polycondensate according to any one of claims 1 to 8, where at least one monomeric compound of the formula (II)
Z¹-(CH₂)₂ₙ₊₁-Z^{1'} (II)
in which
Z¹, Z¹' is ∼C(=O)-Q or ∼CH₂-NCO;
Q is identical or different and is OH, halogen or C₁-C₁₀-alkoxy;
∼ indicates the bond to the group (CH₂)₂ₙ₊₁ and
n is a number ≥ 10,
is polycondensed with at least one di- or polyol or at least one di- or polyamine,
and/or
at least one monomeric compound of the formula (III),
Z²-(CH₂)₂ₙ₊₁-Z^{2'} (III)
in which
Z, Z' is identical or different and is HO-CH₂∼ or H₂N-CH₂∼;
∼ indicates the bond to the group (CH₂)ₙ and
N is a number ≥ 10,
is polycondensed with at least one di- or polycarboxylic acid, a reactive derivative of such a di- or polycarboxylic acid or at least one di-or polyisocyanate.

10. The use of a polycondensate according to any one of claims 1 to 8 in moldings, coatings, foams, films, foils and/or fibers.

11. A molding, coating, foil, foam or fiber comprising one or more polycondensates according to any one of claims 1 to 8.

12. A method for producing saturated or unsaturated α,ω-dicarboxylic acids or esters having in each case 2n + 3 carbon atoms in the acid moiety, where n is ≥ 10, comprising the step of a hydroxy- or alkoxycarbonylation of a mono- or polyunsaturated fatty acid ester having 2n + 2 carbon atoms in the acid moiety, where n is ≥ 10, in the presence of a catalyst comprising at least one palladium compound and at least one phosphane, at a temperature in the range from 85 to 100°C and a pressure from 3 to 80 atm.

13. The method according to claim 12, where the alkoxy compound is a C₂-C₃₆-alcohol.

14. The method according to claim 12 or 13, where the catalyst comprises a palladium(II) salt and 1,2-bis[(di-tert-butylphosphino)methyl]benzene.

15. The method according to any one of claims 12 to 14, where the fatty acid ester is an ester of erucic acid.

## Revendications

1. Produit de polycondensation, contenant un ou plusieurs motifs répétitifs de formule (I),
-Z(CH₂)₂ₙ₊₁-Z'- (I)
dans laquelle les symboles et indices ont les significations suivantes :
Z, Z' identiques ou différents représentent -X-C(=O)∼, -C(=O)-HN-CH₂∼, -C(=O)-O-CH₂∼, -NH-C(=O)-O-CH₂∼, -O-C(=O)-NH-CH₂∼ ;
X est O ou NH ;
∼ indique la liaison au groupe (CH₂)ₙ et
n est un nombre ≥ 10.

2. Produit de polycondensation selon la revendication 1, contenant des motifs répétitifs -X-C(=O)-(CH₂)₂ₙ₊₁-C(=O)-X'- où n≥10 et X, X' = O ou NH.

3. Produit de polycondensation selon la revendication 1, contenant des motifs répétitifs -M-(CH₂)₂ₙ₊₃-M'∼ ou n ≥ 10 et M, M' représentent une fonction amide (-C(=O)-NH∼), ester (-C(=O)-O∼) ou uréthane (-NH-C(=O)-O∼).

4. Polyester selon la revendication 1 ou 2, comportant des motifs répétitifs [{-A¹-OC(=O)-(CH₂)₂ₙ₊₁-C(=O)O)O-}ₓ{-A¹-OC(=O)-A²-C(=O)O-}_{y}] où n ≥ 10 et A¹, A², identiques ou différents, représentent un radical aliphatique, cycloaliphatique, aromatique ou mixte en C₂-C₃₆ et x+y= 1.

5. Polyamide selon la revendication 1 ou 2, comportant des motifs répétitifs [{-A¹-NHC(=O)-(CH₂)₂ₙ₊₁-C(=O)O)NH-}ₓ{-A¹-NHC(=O)-A²-C(=O)NH-}_{y}] où n ≥ 10 représentent un radical aliphatique, cycloaliphatique, aromatique ou mixte en C₂-C₃₆ et x+y= 1.

6. Produit de polycondensation selon la revendication 3 ou 4, dans lequel y = 0.

7. Produit de polycondensation selon l'une quelconque des revendications 1 à 6, où n = 10.

8. Produit de polycondensation selon l'une quelconque des revendications 1 à 7, contenant au moins 1 % en moles d'un ou de plusieurs motifs répétitifs de formule (I).

9. Procédé pour la préparation d'un produit de polycondensation selon l'une quelconque des revendications 1 à 8, dans lequel on soumet à une polycondensation au moins un composé monomère de formule (II),
Z¹-(CH₂)₂ₙ₊₁-Z^{1'} (II)
dans laquelle
Z¹, Z^{1'} représentent ∼C(=O)-Q ou -CH₂-NCO ;
Q le même ou différent est OH, un atome d'halogène ou un groupe alcoxy en C₁-C₁₀ ;
∼ indique la liaison au groupe (CH₂)ₙ₊₁ et
n est un nombre ≥ 10,
avec au moins un diol ou polyol ou au moins une diamine ou polyamine,
et/ou
au moins un composé monomère de formule (III),
Z²-(CH₂)₂ₙ₊₁-Z^{2'} (II)
dans laquelle
Z, Z identiques ou différents représentent HO-CH₂∼ ou H₂N-CH₂∼
∼ indique la liaison au groupe (CH₂)ₙ et
n est un nombre ≥ 10,
avec au moins un acide dicarboxylique ou polycarboxylique, un dérivé réactif d'un tel acide dicarboxylique ou polycarboxylique ou au moins un diisocyanate ou polyisocyanate.

10. Utilisation d'un produit de polycondensation selon l'une quelconque des revendications 1 à 8 dans des pièces moulées, des revêtements, des mousses, des films, des feuilles et/ou des fibres.

11. Corps moulées, revêtements, feuilles, mousses et fibres, contenant un ou plusieurs produits de polycondensation selon l'une quelconque des revendications 1 à 8.

12. Procédé pour la préparation d'acides α,ω-dicarboxyliques ou esters, saturés ou insaturés, ayant chacun 2n+3 atomes de carbone dans le fragment acide, n étant ≥10, comprenant l'étape d'une hydroxy- ou alcoxy-carbonylation d'un ester d'acide gras une ou plusieurs fois insaturé, ayant 2n+2 atomes de carbone dans le fragment acide, n étant ≥10, en présence d'un catalyseur contenant au moins un composé de palladium et au moins un phosphane, à une température dans la plage de 85 à 100 °C et sous une pression de 3 à 80 atm.

13. Procédé selon la revendication 12, le composé alcoxy étant un alcool en C₂-C₃₆.

14. Procédé selon la revendication 12 ou 13, dans lequel le catalyseur contient un sel de palladium(II) et du 1,2-bis-[(di-tert-butylphosphino)méthyl]benzène.

15. Procédé selon l'une quelconque des revendications 12 à 14, dans lequel l'ester d'acide gras est un ester de l'acide érucique.
